# EUROPEAN PATENT SPECIFICATION

(11) **EP 3 798 365 B1**
(45) Date of publication and mention of the grant of the patent: **11.05.2022**
(21) Application number: 20805176.3
(22) Date of filing: 14.04.2020
(51) Int. Cl.: E02D 1/00, G01V 9/00, E02D 1/02

(54) **SEABED STATIC PENETRATION DEVICE AND PENETRATION METHOD BASED ON MARINE OBSERVATION PROBE ROD**
STATISCHE MEERESBODENPENETRATIONSVORRICHTUNG UND PENETRATIONSVERFAHREN AUF BASIS VON SONDENSTAB ZUR SEEBEOBACHTUNG
DISPOSITIF DE PÉNÉTRATION STATIQUE DE FOND MARIN ET PROCÉDÉ DE PÉNÉTRATION BASÉS SUR UNE TIGE DE SONDE D'OBSERVATION MARINE

(30) Priority: 15.05.2019 CN 201910407298
(43) Date of publication of application: 31.03.2021
(73) Proprietor: Ocean University of China, Qingdao, Shandong 266100 (CN)
(72) Inventor: JIA, Yonggang, Qingdao, Shandong 266100 (CN); CHEN, Tian, Qingdao, Shandong 266100 (CN); LIU, Xiaolei, Qingdao, Shandong 266100 (CN); QUAN, Yongzheng, Qingdao, Shandong 266100 (CN); GUO, Xiujun, Qingdao, Shandong 266100 (CN); LIU, Tao, Qingdao, Shandong 266100 (CN); ZHU, Chaoqi, Qingdao, Shandong 266100 (CN)
(74) Representative: Petculescu, Ana-Maria
(86) International application number: PCT/CN2020/084575
(87) International publication number: WO 2020/228464

(56) References cited:
- EP-A1- 3 351 974
- WO-A1-2019/078551
- WO-A1-2019/078551
- CN-A- 108 645 917
- CN-A- 110 117 951
- CN-U- 205 384 247
- CN-U- 208 091 756
- CN-Y- 2 210 149
- JP-U- S4 979 006
- US-B1- 8 641 272

## Description

### TECHNICAL FIELD

The present disclosure relates to the technical field of oceanic engineering geologies and in-situ long-term observations of seabed, in particular to a seabed static penetration device based on an oceanographic observation probe rod and a penetration method thereof.

### BACKGROUD

With the development of the social economy, demands for mineral resources and energy are growing in this country, and the ocean has become a destination for massive wealth mining. An increasing number of offshore engineering structures have been put in service, indicating that the ocean exploitation has entered a new stage of development. It is of great significance for the offshore engineering construction safety by obtaining the physical and mechanical performance parameters of seabed sediments, analyzing factors affecting the stability of seabed sediments, and determining the unstable areas and potential geological tectonic effects, which have become research hot areas at present. Among the above researches, a seabed observation probe rod integrated with various sensing probes, acting as the most direct and accurate means to obtain sediment property parameters, has always been a focus of research in the field of oceanographic observation geology and ocean exploration technologies. According to functions, the current observation probe robs can be categorized as static sounding probe rods, seabed pore-water pressure monitoring probe rods, seabed sediment acoustic characteristic measuring probe rods, and seabed sediment resistivity characteristic measuring probe rods, etc. During usage, all the above long and thin rods are required to be vertically inserted into seabed sediments according to the predetermined requirements to achieve observation purposes, and to minimize the interference to the soil mass. Therefore, penetration methods and technologies of the seabed observation probe rod become the major bottleneck keeping out the observation success, and also a technical difficulty in this field.

At present, the penetration methods of the seabed observation probe rod mainly include two ways, the gravity penetration and the static penetration. Among the two methods, the gravity penetration adopts cable hoisting, by which the probe rod penetrates into the seabed in a free-falling way by its own gravity or adding counterweight. This penetration method is relatively simple, but it is problematic in such as uncontrollable insertion depth and great disturbance to the soil mass. In addition, the penetration position can be out of control during the penetration, which often results in that the probe rod cannot be vertically inserted into the soil mass. Further, deployment in areas with some harder or unknown geology may be relatively risky, perhaps seriously leading to bending or even damage of the probe rod during the penetration into the seabed soil mass, thus affecting its function results. The static penetration of the seabed observation probe rod is mostly implemented by a hydraulic transmission device, and the penetration equipment is deployed on the seabed by cable hoisting to implement the penetration process, wherein the penetration device is fixedly connected with the probe rod, providing a penetration force for the rod through an underwater hydraulic system. This underwater hydraulic transmission mode results in low penetration efficiency due to the resistance caused by fluid flows, and the hydraulic oil therein is prone to leak and pollute the site and its performance can be easily affected by temperature changes, making it not suitable for operating at very high or low temperatures. Moreover, the hydraulic penetration equipment is quite bulky and heavy, which causes great disturbance to soil mass and affects the quality of observation results.

CN 208091756 U discloses a seabed static penetration device based on an oceanographic observation probe rod comprising a probe rod stand, an outer clasp slidably connected with the probe rod stand and a base located at the bottom of the probe rod stand.

### SUMMARY

To make up for the deficiencies of the prior art, the present disclosure provides a seabed static penetration device, which has a stable structure, efficient detection, and a great penetration depth, based on an oceanographic observation probe rod and a penetration method thereof.

The present disclosure is implemented through the following technical schemes:

A seabed static penetration device based on an oceanographic observation probe rod, including a probe rod stand, an outer clasp slidably connected with the probe rod stand, and a base located at the bottom of the probe rod stand, wherein: a probe rod positioning device provided with a latch and an electromagnet and a pressure capping structure slidably connected with the probe rod stand are installed on the outer clasp; the base includes an upper base plate and a lower base plate each provided with a bottom contact switch, and a separating plate between the two on which the electromagnet is installed, wherein the upper base plate is provided with a battery compartment, a driver compartment, and a motor which are connected with each other, and the motor is connected to the pressure capping structure through a driving rope, wherein the pressure capping structure includes a cap, a contact rod provided with a magnet and a control compartment connected with the outer clasp; and a probe rod is correspondingly clamped and fixed between the outer clasp and the base.

According to the present disclosure, a deep water motor is adopted to provide a constant speed control and power to ensure the release speed under water. In addition, a "pressure capping" structure is adopted to apply a downward pressure directly to the "cap" on the top of the probe rod, in order to enable the separation between the release device and the probe rod. Because the entire probe rod is quite long, a guide device both uses a guide rod for direction guidance and a base separation plate to further ensure the guidance. The release device and the pore pressure probe rod are attached to each other by latch and electromagnet without rigid connection, which is convenient for the separation between the probe rod and the release device. After the overall release, by simply lifting the release device as a whole, the probe rod will automatically stay underwater for service, and many functions such as release of the hole pressure probe rod under water, retrieval of the release device, and reloading of the probe rod.

A preferred technical scheme of the present disclosure is:

A vertical rod of the probe rod stand is connected with the base by bolts, anti-deformation rings are uniformly welded all over the periphery of the probe rod stand, and a steel wire arrangement device is disposed around the base to ensure that the probe rod stand does not deform.

The outer clasp is fixed on the probe rod stand by bolts, and consists of a left part and a right part connected by bolts, in order to facilitate the fixation and connection between the outer clasp and the probe rod stand.

The base includes a lower base plate connected by a base support rod, wherein an O-ring plate is arranged at the middle of the lower base plate, a through hole corresponding to the probe rod is arranged on the base support rod to facilitate disassembly and fitting between the probe rod and the base, and the O-ring plate is used for underwater guidance to ensure a proper position of the probe rod during operation.

The pressure capping structure has three guide sheaves installed under the cap and connected with the probe rod stand, wherein the cap has a contact rod installed at the bottom thereof, and an electromagnet is installed on the contact rod to correspond to the bottom contact switch on the upper base plate which can detect a contact rod signal and, if any, stop the motor running; the cap has rope-passing sheaves arranged in the middle to let a rope pass, and a protection bolt arranged at top above the rope-passing sheaves, wherein the rope passes through underneath the protection bolt which mainly functions during the lowering down for restraining the rope within the extend of the cap without escape.

The motor includes a 48V brushless DC motor and a speed retarder positioned in an underwater motor seal compartment, and a motor driver is installed in the underwater driver seal cabin. Both the underwater motor seal compartment and the underwater driver seal compartment are filled with oil for sealing, and they are connected with each other by watertight connectors. The motor is used for providing underwater power by underwater DC and a speed closed loop control, and its speed will not be affected by any external load change.

The O-ring plate has a three-layer structure and an adjustment bolt arranged thereon by which the O-ring plate can adapt to the control compartment at different heights.

A method provided in the present disclosure for seabed static penetration by using the above mentioned penetration device includes the following steps:
(1) Before the penetration of the probe rod, the electromagnet installed on the probe rod positioning device is energized, the latch is opened, and the probe rod positioning device is attached by the electromagnet at this moment; by utilizing the shipborne geological winch on the offshore work vessel, the penetration device is released as a whole to the seabed at a designated site by means of cable hoisting;
(2) After the penetration device touches the bottom, a bottom contact switch is triggered, and at this moment the electromagnet attaching the probe rod positioning device is powered off to have the probe rod positioning device turned on, the motor starts running and drives the driving rope to apply power to the pressure capping structure, enabling the probe rod to perform the penetration;
(3) When the probe rod penetrates to a specific depth, the electromagnet on the separation plate is powered off to end the fixation between the separation plate and the base, and the separation plate is separated from the penetration device by gravity;
(4) As the probe rod goes deeper, the cap moves down until it touches the bottom contact switch on the upper base plate, resulting in that the switch is triggered, the motor stops running, and the penetration is done;
(5) The cable is used for overall retrieval of the penetration device, where the probe rod is separated from the penetration device. After the retrieval of the penetration device is completed, the probe rod remains on the seabed to perform observation tasks.

Among the above steps, in Step (1), during the release of the penetration device, the cable release speed is adjusted to the maximum, and slowed down when the penetration device is lowered to 100m away from the seabed.

In the present disclosure, the penetration depth of the probe rod is capable of measuring deep seabed regardless of the limit of the lifting platform stroke. When multiple probe rods are required for penetration, the penetration method is determined on the basis of the conditions of seabed sediments. If the sediments are loose and soft, multiple probe rods may be penetrated simultaneously, and if the sediments are dense, multiple probe rods can be penetrated in sequence. In this way, it not only achieves a high detection efficiency by simultaneous measurement of multiple seabed parameters by multiple probe rods, but also obtains a large penetration depth and measurement parameters of a greater research value.

The present disclosure has the advantages of a reasonable structural design, a stable performance, a high operation efficiency and a small equipment volume, which not only realizes stable, vertical and constant-speed penetration of the oceanographic probe, but also enables a high penetration efficiency and a large penetration depth, thus ensuring the quality of observation data obtained by the seabed observation probe rod.

### BRIEF DESCRIPTION OF THE DRAWINGS

The present disclosure will be further described with reference to figures below.

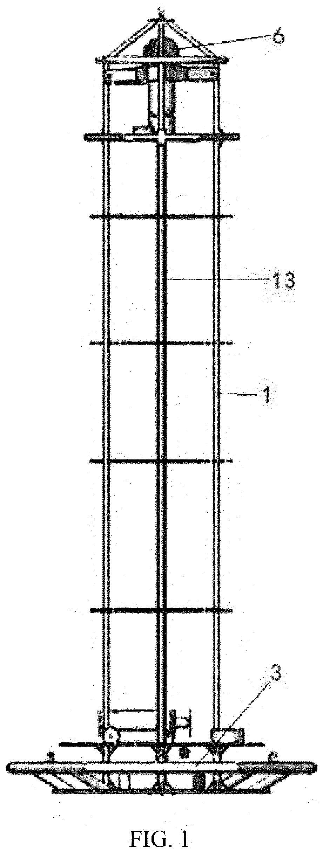

Fig. 1 is a main structure schematic view according to the present invention;

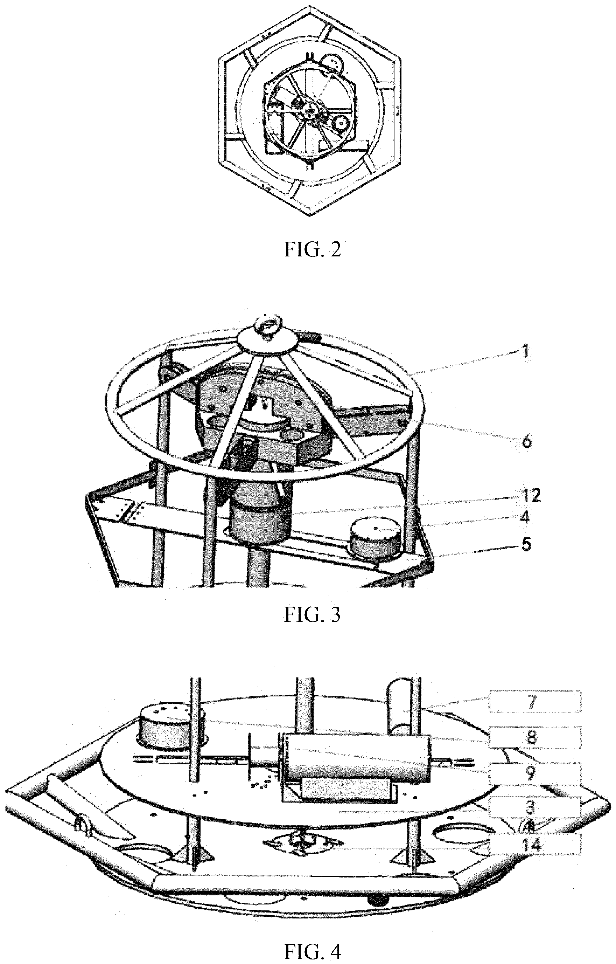

Fig. 2 is a top structure schematic view according to the present invention;
Fig. 3 is a upper structure schematic view according to the present invention;
Fig. 4 is a bottom structure schematic view according to the present invention;
Fig. 5 is a main structure schematic view of the probe rod stand according to the present invention;
Fig. 6 is a top structure schematic view of the probe rod stand according to the present invention;
Fig. 7 is a structure schematic view of the outer clasp according to the present invention;
Fig. 8 is a structure schematic view of the base according to the present invention;
Fig. 9 is a structure schematic view of the O-ring plate according to the present invention;
Fig. 10 is a perspective structure schematic view of the pressuring capping structure according to the present invention;
Fig. 11 is a top structure schematic view of the pressure capping structure according to the present invention;
Fig. 12 is a left structure schematic view according to the present invention;
Fig. 13 is a mechanical structure schematic view of the motor according to the present invention.

In the figures, the reference numbers are 1 for probe rod stand, 2 for outer clasp, 3 for base, 4 for electromagnet, 5 for probe rod positioning device, 6 for pressure capping structure, 7 for battery compartment, 8 for driver compartment, 9 for motor, 10 for cap, 11 for contact rod, 12 for control compartment, 13 for probe rod, 14 for O-ring plate, 15 for protection bolt.

### DETAILED DESCRIPTION

The technical scheme of the present disclosure will be further explained with reference to the drawings and embodiments as follows. It can be understood that the specific embodiments described herein are only for the purpose of illustrating, but not limiting, the present disclosure. In addition, it should be noted that, to facilitate description, not all parts but only those relevant to the present disclosure are shown in the drawings.

Referring to accompanying figures, a seabed static penetration device based on an oceanographic observation probe rod is provided in the present disclosure, including a probe rod stand 1, an outer clasp 2 slidably connected with the probe rod stand 1, and a base 3 located at the bottom of the probe rod stand 1, wherein: a probe rod positioning device 5 provided with a latch and an electromagnet 4 and a pressure capping structure 6 slidably connected with the probe rod stand are installed on the outer clasp 2; the base 3 includes an upper base plate and a lower base plate each provided with a bottom contact switch, and a separating plate between the two on which the electromagnet is installed, wherein the upper base plate is provided with a battery compartment 7, a driver compartment 8, and a motor 9 which are connected with each other, and the motor is connected to the pressure capping structure 6 through a driving rope, wherein the pressure capping structure 6 includes a cap 10, a contact rod 11 provided with a magnet and a control compartment 12 connected with the outer clasp 2; and a probe rod 13 is correspondingly clamped and fixed between the outer clasp 2 and the base 3; a deep water motor is adopted to provide a constant speed control and power, and a driving rope is adopted to pass the power to the pressure capping structure so as to apply a downward pressure directly to the "cap" on the top of the probe rod, enabling the penetration of the probe rod. Each component of the penetration device will be described in detail below.

### (1) Probe rod stand 1

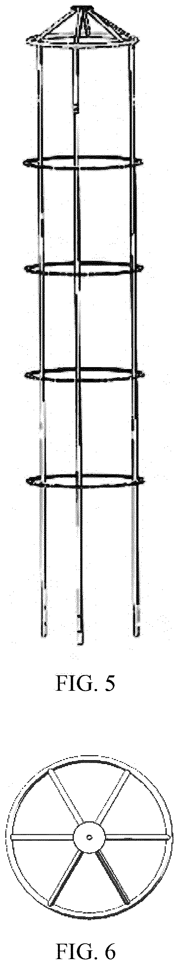

See Figs. 5-6 for the structure of the probe rod stand 1. The probe rod stand 1 mainly functions to support and guide, and adopts three vertical pipes of the probe rod stand 1 as tracks for the cap 10 to slide down in order to ensure the vertical lowering of the probe rod 13. The probe rod stand 1, about 6 meters long, has 4 in total anti-deformation rings (arranged at an interval of 1 meter) welded around its periphery and a steel wire arrangement position reserved on the base 3 thereof, with the steel wire to be installed at the end to further prevent the probe rod stand 1 from tilt and deformation. The vertical rod is connected with the base 3 by bolts, and the vertical rod has holes at the bottom to fit the base 3 for installation, which facilitating the disassembly and installation of the base 3 and the probe rod stand 1, and enabling the convenience of overall transportation.

### (2) Outer clasp 2

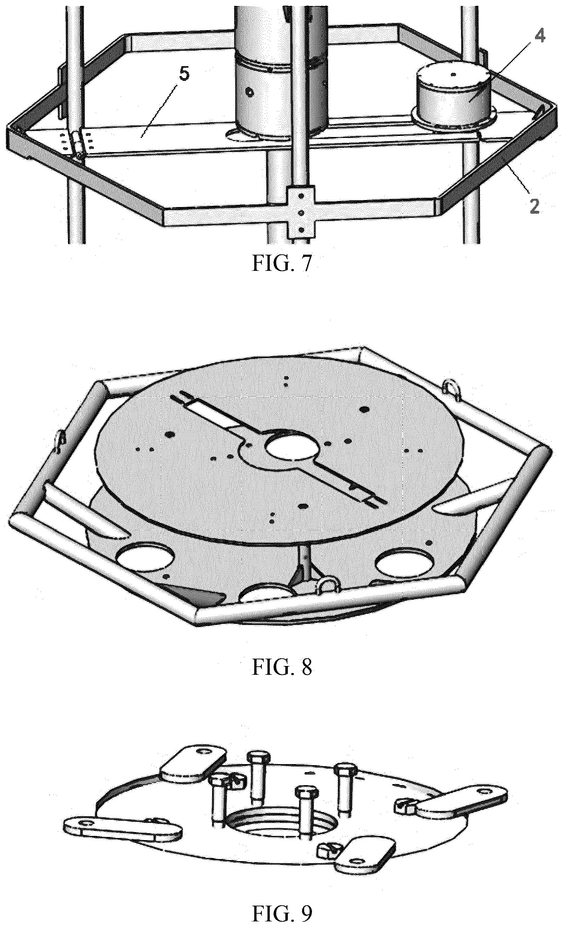

See Fig. 7 for the structure of the outer clasp 2. The outer clasp 2 device mainly functions for the installation and positioning of the probe rod 13. In order to enable the automatic separation between the probe rod 13 and the release device, there is no rigid connection mechanism between them, such that a special mechanism is required for locating an initial position.

The outer clasp 2 is equipped with two ways of fixing, i.e., before launching, the latch is used for fixing, which is beneficial to energy saving and overall installation. Then, before the device enters the water, the electromagnet 4 is energized and the latch is unlatched.

The electromagnet 4 is installed on the outer clasp 2. As the bottom contact switch at the bottom is triggered, the signal controls the electromagnet to be powered off, the switch is turned on, and the probe rod is allowed to lower down. At the same time, the motor 9 starts the operation and the penetration.

The working principle of the outer clasp 2 is to manually pull the probe rod 13 up to the upper part at first, then close the baffle and fix it with a latch.

The outer clasp 2 consists of left and right parts which are connected by bolts and can be simply fixed on the probe rod stand 1 by bolts during installation.

### (3) Base 3

See Figs. 8 for the structure of the base 3. The base 3 adopts a frame structure which both ensures the strength and a volume as small as possible. In addition, it has a steel wire arrangement device disposed around to ensure the steadiness and stability of the entire device.

The upper base plate has mounting positions thereon reserved for devices such as the motor. The upper and lower base plates are each provided with a bottom contact switch, respectively.

The bottom contact switch on the lower base plate is used to detect the bottom contact signal. After the penetration device touches the bottom, the electromagnet is powered off and the motor 9 is turned on.

The bottom switch on the upper base plate is used to monitor the probe rod position. After the probe rod 13 reaches a designated position, the motor 9 stops running.

A through hole is reserved on the base support rod, which fits to and cooperates with the probe rod 13, so as to facilitate the disassembly and installation of the probe rod 13 and the base 3.

The O-ring plate 14 is installed on the base 3, which is used for underwater guidance to ensure a proper working position of the probe rod 13.

When the probe rod 13 is lowered to the bottom, the O-ring plate 14 is pressed down, the plate and the release device fall off, and both the O-ring plate 14 and the probe rod 13 stay underwater.

The O-ring plate 14 consists of three layers, each of which has its own function and the three are integrated as a whole. The O-ring plate has an adjustment bolt arranged thereon by which the O-ring plate can adapt to the control compartment at different heights.

### (4) Pressure capping structure 6

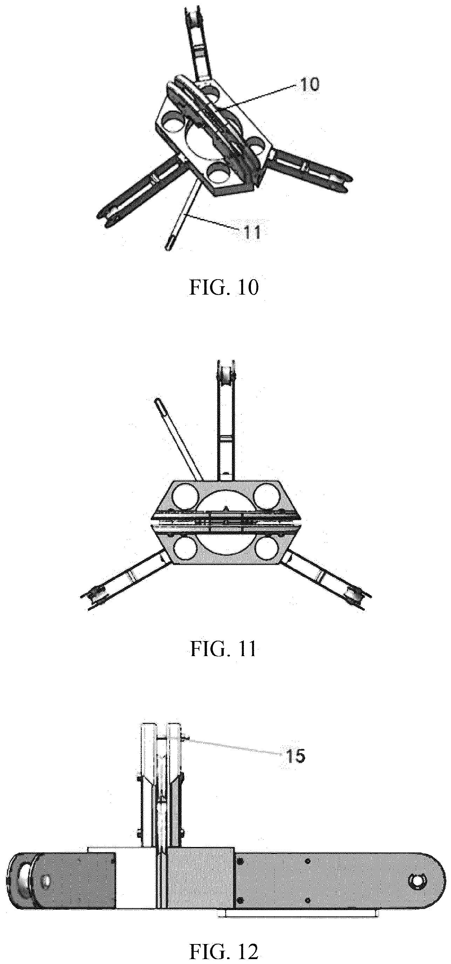

See Figs. 10-12 for the structure of the pressure capping structure 6. The pressure capping structure 6 is mainly used for pressing the probe rod 13 and the control compartment 12 to move them downwards. The cap 10 has three guide sheaves to ensure the stability during the downward sliding, and at the same time useless materials are removed from the main body of the cap 10 to lessen the overall weight.

A contact rod 11 is installed at the bottom of the cap 10, and a magnet is installed on the rod. The bottom contact switch on the base 3 will detect the magnetic signal, and if any signal is detected, the motor stops running. Meanwhile, the contact rod 11 is connected by bolts, so that it can be manually adjusted for placement at any time to ensure the stability of function implementation.

The cap 10 adopts 4 rope-passing sheaves arranged in the middle thereof to ensure a uniform force on the entire rope when the rope passes through. A separate protection bolt 15 is arranged on top of the cap 10, and the rope passes through underneath the guarantee bolt 15 which is mainly used during the lowering down for restraining the rope within the extend of the cap 10 without escape.

### (5) Underwater motor driving system

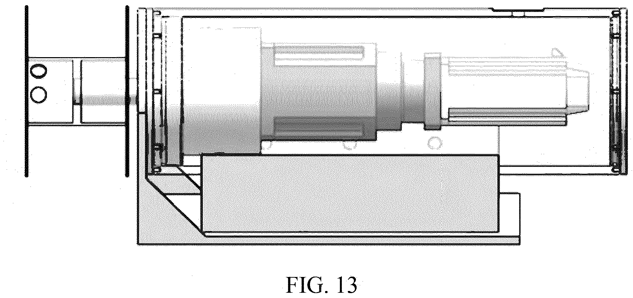

See Fig 13 for the structure of the underwater motor driving system. The motor 9 is used for driving the underwater equipment.

The motor 9 consists of a 48V brushless DC motor, a speed retarder, and an underwater motor seal compartment, etc.

The interior of the underwater motor seal compartment is filled with oil for sealing to ensure the balance between the internal and external pressures when the penetration device is operating in deep water.

The underwater motor seal compartment and the underwater driver seal compartment are connected with each other by watertight connectors which facilitate disassembly and installation, and also facilitate transportation and installation.

The underwater motor seal compartment can be installed on the base 3 which facilitates positioning and installation.

### (6) Underwater control system

The underwater control system includes a power supply system and a cap release system.

This part includes the battery seal compartment, and the electromagnet seal compartment, etc.

The seal compartment guarantees the elements and components therein can be used in water, and has watertight connectors reserved therein. During installation, the compartment only needs to be fixed in a proper position, and then the watertight connectors can be installed.

The embodiment of the present disclosure provides a seabed static penetration method based on an oceanographic observation probe rod, including the following steps:

Before the penetration of the probe rod 13, the electromagnet 4 installed on the probe rod positioning device 5 is energized, the latch is opened, and the probe rod positioning device 5 is attached by the electromagnet 4 at this moment. By utilizing the shipborne geological winch on the offshore work vessel, the penetration device is released as a whole to the seabed at a designated site by means of cable hoisting, and during the release of the penetration device, the cable release speed is adjusted to the maximum and then slowed down when the penetration device is lowered to 100m away from the seabed.

After the penetration device touches the bottom, the bottom contact switch is triggered, and at this moment the electromagnet 4 attaching the probe rod positioning device 5 is powered off to have the probe rod positioning device 5 turned on, the motor 9 starts running and drives the driving rope to apply power to the pressure capping structure 6, enabling the probe rod 13 to perform the penetration at a constant speed of 0.02m/s. When the probe rod 13 penetrates to a specific depth, the electromagnet on the separation plate is powered off to end the fixation between the separation plate and the base 3, and the separation plate is separated from the penetration device by gravity. As the probe rod 13 goes deeper, the cap 10 moves down to a certain depth until it touches the second bottom contact switch, resulting in that the second switch is triggered, the motor 9 stops running at the moment, the penetration of the penetration device is done, and the probe rod penetrates into the seabed.

After the penetration of the probe rod 13, a cable is used for overall retrieval of the penetration device, where the probe rod 13 is separated from the penetration device. After the retrieval of the penetration device is completed, the probe rod 13 remains on the seabed to perform observation tasks.

The above mentioned penetration device can make the probe rod 13 vertically penetrate into the seabed soil mass at a constant speed. This penetration mode ensures the penetration quality of the probe rod 13, a great penetration depth, reliable measurement parameters, and a greater value in scientific research and engineering application. Moreover, the penetration device can meet the penetration requirements of the probe rod 13 of various sizes, and achieve a high deployment efficiency of seabed observation equipment. In addition, the penetration speed and depth of the probe rod 13 can be adjusted according to different practical requirements, which will bring significant convenience for ocean observation events.

## Claims

1. A seabed static penetration device comprising an oceanographic observation probe rod, comprising a probe rod stand (1), an outer clasp (2) slidably connected with the probe rod stand (1), and a base (3) located at the bottom of the probe rod stand (1), **characterized by** : a probe rod positioning device (5) provided with a latch and an electromagnet (4) and a pressure capping structure (6) slidably connected with the probe rod stand (1) are installed on the outer clasp (2); the base (3) comprises an upper base plate and a lower base plate each provided with a bottom contact switch, and a separating plate between the two on which the electromagnet is installed, wherein the upper base plate is provided with a battery compartment (7), a driver compartment (8), and a motor (9) which are connected with each other, and the motor is connected to the pressure capping structure (6) through a driving rope, wherein the pressure capping structure (6) comprises a cap (10), a contact rod (11) provided with a magnet and a control compartment (12) connected with the outer clasp (2); and a probe rod (13) is correspondingly clamped and fixed between the outer clasp (2) and the base (3).

2. The seabed static penetration device according to claim 1, wherein: the vertical probe rod rod of the probe rod stand (1) is connected with the base (3) by bolts, anti-deformation rings are uniformly welded all over the periphery of the probe rod stand (1), and a steel wire arrangement device is disposed around the base (3).

3. The seabed static penetration device according to claim 1, wherein: the outer clasp (2) is fixed on the probe rod stand (1) by bolts, and consists of a left part and a right part connected with each other by bolts.

4. The seabed static penetration device according to claim 1, wherein: the base (3) comprises a lower base plate connected by a base support rod, wherein an O-ring plate (14) is installed at the middle of the lower base plate, and a through hole corresponding to the probe rod (13) is arranged on the base support rod.

5. The seabed static penetration device according to claim 1, wherein: the pressure capping structure (6) has three guide sheaves installed under the cap (10) and connected with the probe rod stand (1), wherein the cap (10) has a contact rod (11) installed at the bottom thereof, and an electromagnet is installed on the contact rod (11) to correspond to the bottom contact switch on the upper base plate (3); the cap (10) has rope-passing sheaves arranged in the middle to let a rope pass, and a protection bolt (15) arranged at top above the rope-passing sheaves.

6. The seabed static penetration device according to claim 1, wherein: the motor (9) comprises a 48V brushless DC motor and a speed retarder positioned in an underwater motor seal compartment, and a motor driver is installed in the underwater driver seal cabin, wherein both the underwater motor seal compartment and the underwater driver seal compartment are filled with oil for sealing, and they are connected with each other by watertight connectors.

7. The seabed static penetration device to claim 4, wherein: the O-ring plate (14) has a three-layer structure and an adjustment bolt arranged on the O-ring plate (14).

8. A method for seabed static penetration making use of the penetration device according to claim 1, comprising the steps of : (1) before the penetration of the probe rod, the electromagnet installed on the probe rod positioning device according to claim 1 is energized, the latch is opened, and the probe rod positioning device is attached by the electromagnet at this moment; utilizing a shipborne geological winch on an offshore work vessel, the penetration device is released as a whole to the seabed at a designated site by means of cable hoisting; (2) after the penetration device touches the bottom, a bottom contact switch is triggered, and at this moment the electromagnet attaching the probe rod positioning device is powered off to have the probe rod positioning device turned on, the motor starts running and drives the driving rope to apply power to the pressure capping structure, enabling the probe rod to perform the penetration; (3) when the probe rod penetrates to a specific depth, the electromagnet on the separation plate is powered off to end the fixation between the separation plate and the base, and the separation plate is separated from the penetration device by gravity; (4) as the probe rod goes deeper, the cap moves down until it touches the bottom contact switch on the upper base plate, resulting in that the switch is triggered, the motor stops running, and the penetration is done; (5) the cable is used for overall retrieval of the penetration device, where the probe rod is separated from the penetration device; after the retrieval of the penetration device is completed, the probe rod remains on the seabed to perform observation tasks.

9. The method for seabed static penetration according to claim 8, wherein: in Step (1), during the release of the penetration device, the cable release speed is adjusted to the maximum, and then slowed down when the penetration device is lowered to 100m away from the seabed.

## Patentansprüche

1. Statische Meeresbodenpenetrationsvorrichtung, umfassend einen ozeanographischen Beobachtungssondenstab, umfassend einen Sondenstabständer (1), eine Außenklammer (2), die verschiebbar mit dem Sondenstabständer (1) verbunden ist, und eine Basis (3), die an der Unterseite des Sondenstabständers (1) angeordnet ist, **dadurch gekennzeichnet, dass** eine Sondenstabpositionierungsvorrichtung (5), die mit einem Riegel und einem Elektromagneten (4) versehen ist, und eine Druckkappenstruktur (6), die verschiebbar mit dem Sondenstabständer (1) verbunden ist, an der Außenklammer (2) installiert sind; wobei die Basis (3) eine obere Basisplatte und eine untere Basisplatte, die jeweils mit einem unteren Kontaktschalter versehen sind, und eine Trennplatte zwischen den beiden umfasst, an der der Elektromagnet installiert ist, wobei die obere Basisplatte mit einer Batterieaufnahme (7), einer Antriebsaufnahme (8) und einem Motor (9) versehen ist, die miteinander verbunden sind, und der Motor durch ein Antriebsseil mit der Druckkappenstruktur (6) verbunden ist, wobei die Druckkappenstruktur (6) eine Kappe (10), einen Kontaktstab (11), der mit einem Magneten versehen ist, und eine Steuerungsaufnahme (12) umfasst, die mit der Außenklammer (2) verbunden ist; und ein Sondenstab (13) entsprechend zwischen der Außenklammer (2) und der Basis (3) eingeklemmt und fixiert ist.

2. Statische Meeresbodenpenetrationsvorrichtung nach Anspruch 1, wobei: der vertikale Sondenstab des Sondenstabständers (1) durch Schrauben mit der Basis (3) verbunden ist, Antiverformungsringe gleichmäßig um den gesamten Umfang des Sondenstabständers (1) geschweißt sind und eine Stahldrahtanordnungsvorrichtung um die Basis (3) herum angeordnet ist.

3. Statische Meeresbodenpenetrationsvorrichtung nach Anspruch 1, wobei: die Außenklammer (2) durch Schrauben am Sondenstabständer (1) fixiert ist und aus einem linken Teil und einem rechten Teil besteht, die durch Schrauben miteinander verbunden sind.

4. Statische Meeresbodenpenetrationsvorrichtung nach Anspruch 1, wobei: die Basis (3) eine untere Basisplatte umfasst, die mit einem Basisträgerstab verbunden ist, wobei eine O-Ring-Platte (14) in der Mitte der unteren Basisplatte installiert ist und ein Durchgangsloch, das mit dem Sondenstab (13) übereinstimmt, an dem Basisträgerstab angeordnet ist.

5. Statische Meeresbodenpenetrationsvorrichtung nach Anspruch 1, wobei: die Druckkappenstruktur (6) drei Führungsscheiben aufweist, die unter der Kappe (10) installiert sind und mit dem Sondenstabständer (1) verbunden sind, wobei die Kappe (10) einen Kontaktstab (11) aufweist, der an ihrer Unterseite installiert ist, und ein Elektromagnet an dem Kontaktstab (11) in Entsprechung zu dem unteren Kontaktschalter an der oberen Basisplatte (3) installiert ist; wobei die Kappe (10) eine Seilführungsscheibe aufweist, die in der Mitte angeordnet ist, um ein Seil vorbeizuführen, und eine Schutzschraube (15) an der Oberseite über den Seilführungsscheiben angeordnet ist.

6. Statische Meeresbodenpenetrationsvorrichtung nach Anspruch 1, wobei: der Motor (9) einen bürstenlosen 48-V-Gleichstrommotor und einen Verzögerer umfasst, die in einer Unterwassermotorabdichtungsaufnahme angeordnet sind, und ein Motorantrieb in der Unterwasserantriebsabdichtungsaufnahme installiert ist, wobei die Unterwassermotorabdichtungsaufnahme und die Unterwasserantriebsabdichtungsaufnahme zum Abdichten mit Öl gefüllt sind und durch wasserfeste Verbinder miteinander verbunden sind.

7. Statische Meeresbodenpenetrationsvorrichtung nach Anspruch 4, wobei: die O-Ring-Platte (14) eine dreilagige Struktur und eine Stellschraube aufweist, die an der O-Ring-Platte (14) angeordnet ist.

8. Verfahren zur statischen Meeresbodenpenetration unter Verwendung der Penetrationsvorrichtung nach Anspruch 1, folgende Schritte umfassend: (1) wobei vor der Penetration des Sondenstabs der Elektromagnet, der an der Sondenstabpositionierungsvorrichtung nach Anspruch 1 installiert ist, mit Strom versorgt wird, der Riegel geöffnet wird und die Sondenstabpositionierungsvorrichtung auf diese Weise durch den Elektromagneten befestigt wird; wobei mit einer schiffsgetragenen geologischen Winde an einem Offshore-Arbeitsschiff die Penetrationsvorrichtung an einem festgelegten Standort als Ganzes mittels Kabelhebezeug zum Meeresboden abgelassen wird; (2) wenn die Penetrationsvorrichtung den Boden berührt hat, ein Bodenkontaktschalter ausgelöst wird und der Elektromagnet, der die Sondenstabpositionierungsvorrichtung befestigt, ausgeschaltet wird, um die Sondenstabpositionierungsvorrichtung einzuschalten, der Motor startet und das Antriebsseil antreibt, um Kraft auf die Druckkappenstruktur auszuüben, sodass der Sondenstab die Penetration ausführen kann; (3) wenn die Sondenstab bis auf eine bestimmte Tiefe eingedrungen ist, der Elektromagnet an der Trennplatte ausgeschaltet wird, um die Fixierung zwischen der Trennplatte und der Basis zu beenden, und die Trennplatte mittels Schwerkraft von der Penetrationsvorrichtung getrennt wird; (4) sich die Kappe mit zunehmender Tiefe des Sondenstabs abwärts bewegt, bis sie den Bodenkontaktschalter an der oberen Basisplatte berührt, wodurch der Schalter ausgelöst wird, der Motor anhält und die Penetration erfolgt; (5) das Kabel zur gesamten Rückholung der Penetrationsvorrichtung verwendet wird, wobei der Sondenstab von der Penetrationsvorrichtung getrennt wird; wobei nach dem Abschluss der Rückholung der Penetrationsvorrichtung der Sondenstab auf dem Meeresboden zurückbleibt, um Beobachtungsaufgaben auszuführen.

9. Verfahren zur statischen Meeresbodenpenetration nach Anspruch 8, wobei: in Schritt (1) während der Freigabe der Penetrationsvorrichtung die Kabelfreigabegeschwindigkeit auf das Maximum eingestellt wird und dann reduziert wird, wenn die Penetrationsvorrichtung bis auf 100 m vom Meeresboden entfernt abgesenkt wurde.

## Revendications

1. Un dispositif de pénétration statique de fonds marins comprenant une tige de sonde d'observation océanographique, comprenant un statif de tige de sonde (1), un fermoir externe (2) connecté de façon coulissante au statif de tige de sonde (1), et une base (3) située au bas du statif de tige de sonde (1), **caractérisé en ce qu'**un dispositif de positionnement de tige de sonde (5) muni d'un verrou et d'un électroaimant (4) et une structure de bouchonnage sous pression (6) connectée de façon coulissante au statif de tige de sonde (1) sont installés sur le fermoir externe (2) ; la base (3) comprend une plaque de base supérieure et une plaque de base inférieure munies chacune d'un interrupteur de contact de fond, et d'une plaque de séparation entre les deux sur laquelle l'électroaimant est installé, dans lequel la plaque de base supérieure est munie d'un compartiment de batterie (7), un compartiment d'entraînement (8), et un moteur (9) mutuellement connectés, et le moteur est connecté à la structure de bouchonnage sous pression (6) via une courroie d'entraînement, dans lequel la structure de bouchonnage sous pression (6) comprend un couvercle (10), une tige de contact (11) munie d'un aimant et un compartiment de commande (12) connecté au fermoir externe (2) ; et une tige de sonde (13) est serrée et fixée de façon correspondante entre le fermoir externe (2) et la base (3).

2. Un dispositif de pénétration statique de fonds marins selon la revendication 1, dans lequel : la tige de sonde verticale du statif de tige de sonde (1) est connectée à la base (3) par des boulons, des anneaux anti-déformation sont soudés de façon uniforme sur toute la périphérie du statif de tige de sonde (1), et un dispositif d'agencement de câble d'acier est placé autour de la base (3).

3. Un dispositif de pénétration statique de fonds marins selon la revendication 1, dans lequel : le fermoir externe (2) est fixé sur le statif de tige de sonde (1) par des boulons, et consiste en une partie gauche et une partie droite mutuellement connectées par des boulons.

4. Un dispositif de pénétration statique de fonds marins selon la revendication 1, dans lequel : la base (3) comprend une plaque de base inférieure connectée par une tige de support de base, dans laquelle une plaque de joint torique (14) est installée au milieu de la plaque de base inférieure, et un trou traversant correspondant à la tige de sonde (13) est agencé sur la tige de support de base.

5. Un dispositif de pénétration statique de fonds marins selon la revendication 1, dans lequel : la structure de bouchonnage sous pression (6) est munie de trois poulies de guidage installées sous le couvercle (10) et est connectées au statif de tige de sonde (1), dans lequel le couvercle (10) est muni d'une tige de contact (11) installée à sa partie inférieure, et un électroaimant est installé sur la tige de contact (11) afin de correspondre à l'interrupteur de contact de fond sur la plaque de base supérieure (3) ; le couvercle (10) est muni de poulies passe-câbles agencés au milieu afin de laisser passer un câble, et un boulon de protection (15) agencé au-dessus des poulies passe-câbles.

6. Un dispositif de pénétration statique de fonds marins selon la revendication 1, dans lequel : le moteur (9) comprend un moteur sans balais à courant continu 48V et un ralentisseur de vitesse positionné dans un compartiment hermétique de moteur sous-marin, et un pilote de moteur est installé dans la cabine hermétique du pilote sous-marin, dans lequel le compartiment hermétique de moteur sous-marin et le compartiment hermétique de pilote sous-marin sont remplis d'huile pour le scellement, et sont mutuellement connectés par des connecteurs étanches.

7. Un dispositif de pénétration statique de fonds marins selon la revendication 4, dans lequel : la plaque de joint torique (14) est munie d'une structure à trois couches et d'un boulon d'ajustement agencé sur la plaque de joint torique (14).

8. Un procédé de pénétration statique de fonds marins utilisant le dispositif de pénétration statique de fonds marins selon la revendication 1, comprenant les étapes suivantes : (1) avant pénétration de la tige de sonde, l'électroaimant installé sur le dispositif de positionnement de tige de sonde selon la revendication 1 est sous tension, le verrou est ouvert, et le dispositif de positionnement de tige de sonde est retenu par l'électroaimant à ce moment ; un treuil géologique embarqué sur un navire de travail en haute mer est utilisé, le dispositif de pénétration est libéré entièrement sur le fond marin d'un site désigné par moyen de câble de levage ; (2) lorsque le dispositif de pénétration touche le fond, l'interrupteur de contact de fond est mis en marche, et à ce moment l'électroaimant retenant le dispositif de positionnement de tige de sonde est mis à l'arrêt afin que le dispositif de positionnement de tige de sonde soit mis en marche, le moteur commence à fonctionner et entraîne l'application d'une puissance sur la structure de bouchonnage sous pression par la courroie d'entraînement, permettant à la tige de sonde d'effectuer la pénétration ; (3) lorsque la tige de sonde pénètre une profondeur spécifique, l'électroaimant sur la plaque de séparation est mis à l'arrêt afin de faire cesser la fixation entre la plaque de séparation et la base, et la plaque de séparation est séparée du dispositif de pénétration par gravité ; (4) tandis que la tige de sonde s'enfonce, le couvercle se déplace vers le bas jusqu'à ce qu'il touche l'interrupteur de contact de fond sur la plaque de base supérieure, engendrant le déclenchement de l'interrupteur, le moteur cesse de fonctionner, et la pénétration est achevée ; (5) le câble est utilisé pour le retrait total du dispositif de pénétration, tandis que la tige de sonde est séparée du dispositif de pénétration ; une fois le retrait du dispositif de pénétration achevé, la tige de sonde reste sur le fond marin pour effectuer des tâches d'observation.

9. Un procédé de pénétration statique de fonds marins selon la revendication 8, dans lequel : lors de l'étape (1), pendant la libération du dispositif de pénétration, la vitesse de libération du câble est réglée au maximum, puis ralentie lorsque le dispositif de pénétration est abaissé à 100 m de distance du fond marin.
